# EUROPEAN PATENT APPLICATION

(11) **EP 1 459 676 A1**
(43) Date of publication of application: **22.09.2004**
(21) Application number: 04101139.6
(22) Date of filing: 19.03.2004
(51) Int. Cl.: A61B 3/103, A61B 3/107

(54) **Refractometer and control method of said refractometer**

(30) Priority: 21.03.2003 IT MI20030569
(71) Applicant: Ligi Tecnologie Medicali S.p.A., 74100 Taranto (IT)
(72) Inventor: D'Ippolito, Giuseppe, 74100, Taranto (IT); Lipari, Eugenio, 98021, Ali' Terme (IT)
(74) Representative: Jorio, Paolo, Dr. Ing.

(57) **Abstract**

A refractometer includes a first light source (6), providing a light beam (F) having an essentially spot cross section, and a pointing device (7) of the beam (F), to direct the beam (F) towards a point (P) of incidence selectable on a cornea (2) of an eye (1) of a patient; the refractometer is also provided with an image acquisition device (14) to acquire at least a first image of a retinal projection (R) of the beam (F); a control unit (15), connected to the image acquisition device (14), to determine a deviation (S) of the retinal projection (R) with respect to a pre-determined region of reference (4a) of the eye; an optical positioning device (12) of the beam (F), controlled by the control unit (15) to modify a direction (D) of incidence of the beam (F) on the point (P) of incidence so as to minimize the deviation (S).

## Description

The present invention relates to a refractometer and to a control method of said refractometer.

As is known, the treatment of some visual defects has in recent years undergone extremely important improvements thanks to the possibility of operating by means of devices for photoablative surgery, able to perform point-by-point modification of the surface of the cornea of the patient. In the presence of these visual defects, which may occur due to imperfections not only of the frontal surface of the cornea, but also of any part of the system of lenses forming the eye, the patient has a refractive error: the rays impinging on the corneal surface are dispersed on the retina as a function of the refractive defect, rather than being focused in a single point (center of vision, which in an eye falling within the normal range coincides with the region of maximum sensitivity of the retina or fovea). More precisely, a ray projected on a point of the corneal surface parallel to the optical axis impinges on a region of the retina which is different to the fovea. By means of the devices for photoablative surgery it is possible to locally modify the refractive properties of the cornea, so as to compensate with precision the refractive error of the eye point by point and obtain clear vision.

However, it is evident that the efficacy of the photoablative surgery operations is greatly influenced by the precision in determining the corneal surface and, above all, the local refractive error. For this purpose various devices have been produced, such as wavefront analyzers and ray-tracing systems, which however have limits.

Wavefront analyzers have a spot light source, which projects a light beam towards the eye of the patient with an incident wavefront, and a matrix of lenses facing the eye, to collect a wavefront reflected by the retina. The lenses are arranged on a plane and are paired with respective portions of the cornea on a one-to-one basis; in other words, each lens collects only the part of the reflected wavefront that emerges through the corresponding portion of cornea and is utilized to determine the refractive error of that specific portion of cornea. Nonetheless, when the eye is affected by important refractive errors, there is no longer correspondence between lenses and portions of cornea. In fact, on the surface of the cornea and inside the eye the rays may be deflected to such an extent that parts of the reflected wavefront emerging from distinct portions of cornea are collected by the same lens. It is therefore evident that measurement of the refractive error in these conditions is not reliable.

Ray-tracing systems are equipped with a light source, with an image acquisition system and with a processing unit. A light beam with an essentially spot section is emitted by the source and directed towards a pre-determined point of the cornea. The image acquisition and processing system detects the position of the projection on the retina of the incident ray (retinal projection of the incident ray). As mentioned hereinbefore, when there is a refractive defect, the retinal projections of parallel incident rays on different points of the corneal surface do not coincide. In fact, with respect to the center of vision the retinal projection of each incident ray has a relative deflection, which is used as index of the refraction defect: the greater the relative deviation of the retinal projection of the incident ray, the greater the refractive error associated with the point of the cornea on which the incident ray is projected. In general, the relative deviation is assumed to depend on the refractive error essentially according to a linear relation. Nonetheless, in this way an approximation that is often unacceptable is introduced. In fact, even by using more complex relations than the simple proportionality it is impossible to take account of the fact that the refraction defects occur for different reasons and that, therefore, the relation existing between relative deviation and refractive error may vary from case to case. For example, a certain relative deviation may be determined either by a defect of the outer corneal surface or by an irregularity of the inner corneal surface or of the crystalline lens. In other words, measurements of the refractive error performed using traditional ray-tracing systems, which are based on the use of pre-defined relations between refractive error and relative deviation, are not always reliable, due to the fact that these relations cannot be considered acceptable in all cases. The object of the present invention is to provide a refractometer and a control method of said refractometer that overcome the drawbacks described.

According to the present invention a refractometer is provided, comprising a first source of light, supplying a light beam with an essentially spot cross section, and optical pointing means of the beam, for directing the beam towards a point of incidence selectable on a cornea of an eye of a patient;
characterized in that it comprises:
image acquisition means for acquiring at least a first image of a retinal projection of the beam;
a control unit, connected to said image acquisition means, for determining a deviation of the retinal projection with respect to a pre-determined reference region of the eye;
optical directing means of the beam, controlled by said control unit for modifying a direction of incidence of the beam on the point of incidence so as to minimize said deviation.

In this way, the direction of incidence of the beam may be modified on the basis of the deviation, in particular until the retinal projection essentially coincides with the reference region of the eye. The difference between the final direction of incidence and the initial direction of incidence is indicative of the local refractive error and takes account of the overall effect of all the defects present along the path of the beam between the point of incidence on the cornea and the retinal projection. The device according to the invention, in practice, provides a measurement of the local refractive error which is essentially free from approximations and therefore precise and reliable, even when there are severe refractive defects.

According to a further aspect of the invention, the control unit comprises a first image processing unit, for determining a first position of the retinal projection with respect to a pre-determined reference system, on the basis of the first image.

According to a further aspect of the invention, the control unit comprises a second image processing unit, for determining a second position of the reference region with respect to said reference system, on the basis of a second image.

In this way, acquisition of images of the reference region and of the retinal projection allows the deviation to be evaluated with high precision; in particular, errors in evaluating the position of the reference region are avoided. The images acquired may in fact be aligned exactly utilizing a common reference system. Advantageously, the fovea or, alternatively, the center of vision, may be selected as reference region. In particular, the use of the fovea as reference retinal region allows optimization of the quality of vision.

Moreover, the measurement of the deviation, practically without error margin, provides decisive information to control the optical positioning means of the beam efficaciously and to perform measurement in an extremely short interval of time. The risks of error due to movements of the eye and fatigue of the patient are thus drastically reduced.

According to the invention, a control method of a refractometer comprising the steps of:
activating a light source, to produce a light beam having an essentially spot cross section; and
controlling pointing means of the light beam, to direct the light beam towards a point of incidence selectable on a cornea of an eye of a patient;
characterized in that it comprises the steps of:
acquiring at least a first image of a retinal projection of the beam;
supplying said first image to a control unit, to determine a deviation of the retinal projection with respect to a pre-determined reference region of the eye;
controlling optical directing means of the beam, for modifying a direction of incidence of the beam on the point of incidence so as to minimize said deviation.

For a better understanding of the invention, an embodiment is now described purely as a non-limiting example and with reference to the accompanying drawings, wherein:
- Figure 1 is a perspective view of an eye of a patient and shows a system of axes of reference;
- Figure 2 is a partial top plan view of the eye in Figure 1;
- Figure 3 is a partial side elevation of the eye in Figure 1;
- Figure 4 is a block diagram relative to a device for point by point determination of the ocular refractive error according to the present invention;
- Figure 5 is a schematic view of a block of the diagram in Figure 4;
- Figure 6 is an enlarged side elevation of the eye in Figure 1;
- Figure 7 is an enlarged front elevation of an inner part of the eye in Figure 1; and
- Figure 8 is a more detailed block diagram of a part of the diagram in Figure 4.

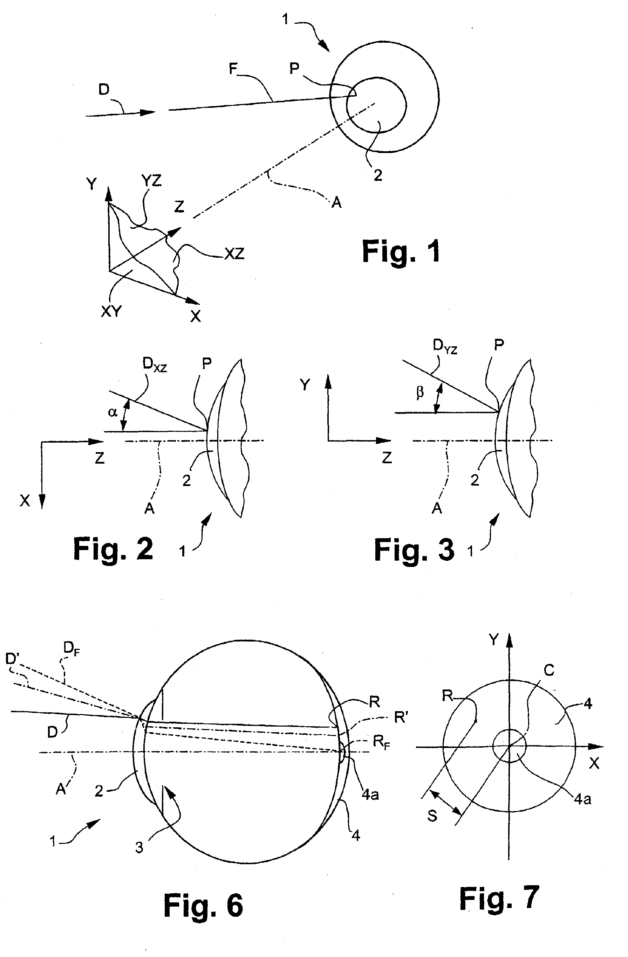

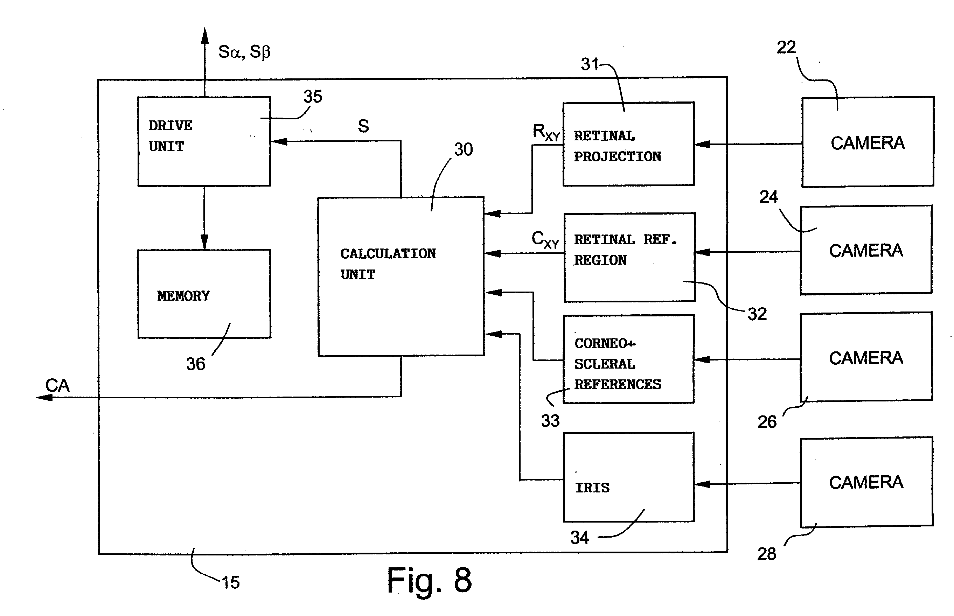

Hereinafter in the description, a system of Cartesian orthogonal reference axes X, Y, Z will be utilized, as shown in Figures 1 to 4, which also show an eye 1 of a patient, having a cornea 2 and an optical axis A. In particular, the axis Z is parallel to the optical axis A (in this case coincident), while the axes X, Y are perpendicular thereto. The axes X, Y, Z define three planes XY, XZ, YZ perpendicular to one another; moreover, the plane XY is perpendicular to the optical axis A of the eye 1 of the patient. Figures 6 and 7 also indicate an iris 3, a retina 4 of the eye 1 and a reference retinal region 4a, in this case the fovea, which is the region of maximum sensitivity of the retina 4.

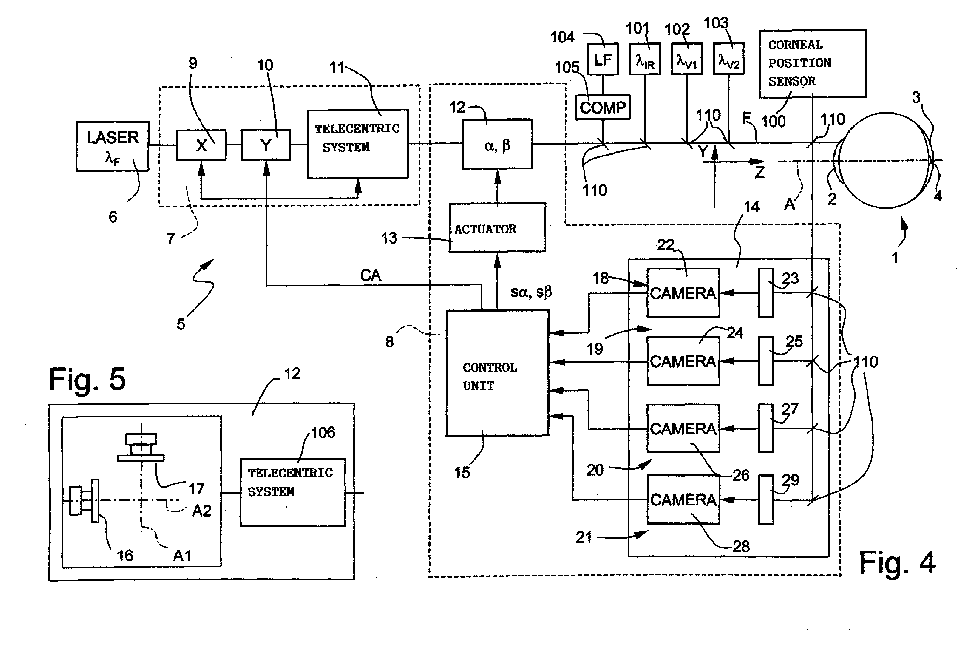

Figure 4 schematically shows a refractometer for point by point determination of the ocular refractive error, indicated as a whole with 5. The refractometer 5 comprises a principal laser source 6, which emits a monochromatic light beam F with a pre-determined wavelength λ_{F} and an essentially spot cross section, a pointing device 7 of the beam F, an angular directing device 12 of the beam F, that can be actuated by means of an actuator 13, an image acquisition device 14 and a logic control unit 15, connected to the image acquisition device 14, to the actuator 13 and to the pointing device 7. The refractometer 5 is also equipped with a corneal position sensor 100, also connected to the control unit 15, an auxiliary source 101 of infrared light, two auxiliary sources 102, 103 of visible light and a fixation source 104, coupled with an optical compensator 105 and supplying a fixation light to align the optical axes A of the eye 1 with the axis Z. In particular, the optical compensator 105 can be controlled for compensating an average refractive error of the eye 1, which may be evaluated with any known investigation method.

The pointing device 7 of the beam F is positioned downstream of a shutter, known and not shown herein, which controls the emission of light by the source 6. In detail, the pointing device 7 comprises a pair of mirrors 9, 10, and a telecentric system of lenses 11. The mirrors 9, 10 are positionable and cooperate with the telecentric system of lenses 11, to direct the beam F toward a point P selectable on the cornea 2 of the eye 1. In practice, the pointing device 7 allows translating the beam F parallel to the axis X and the axis Y and, therefore, selecting the point P of incidence on the cornea; in particular, the beam F may be positioned so as to scan the entire outer surface of the cornea 2. The corneal position sensor 100, per se known, associated with the pointing device 7 and cooperating with the logic control unit 15, allows the position of the point P of incidence of the beam F to be verified; if the point P is not in the desired position, the logic control unit 15 acts on the pointing device 7 to correct it.

As shown in Figure 5, the angular directing device 12 comprises a pair of deflection mirrors 16, 17 and a telecentric system of lenses 106. The deflection mirrors 16, 17 position the beam F according to a pre-determined initial direction D of incidence. Preferably, the initial direction D of incidence is selected taking account of the average refractive error of the eye 1; alternatively, the initial direction D of incidence is parallel to the axis Z. The deflection mirrors 16, 17 are also positionable about respective axes A1, A2 by means of the actuator 13, to modify the initial direction D of incidence of the beam F on the point P of incidence of the cornea 2 in response to commands supplied by the logic control unit 15. In greater detail, the orthogonal projections D_{xz}, D_{YZ} of the axis of the beam F on the planes XZ, YZ form, with the axis Z, a first and, respectively, a second angle of incidence α, β, which define the direction D of incidence of the beam F, as shown in Figures 2 and 3. Positioning of the deflection mirrors 16, 17 determines the values of the first angle of incidence α, in the plane XZ, and of the second angle of incidence β, in the plane YZ; consequently, the direction of incidence of the beam F on the point P of the cornea 2 is also determined. The telecentric system of lenses 106 operates so as to maintain the angle of incidence of the beam F on the cornea 2 notwithstanding any movements of the cornea 2 along the axis Z.

The image acquisition device 14 comprises a line 18 for the acquisition of an image of a retinal projection R of the beam F (or of the point of the retina 4 on which the beam F is incident after having crossed the front surface of the cornea 2 in the point P, Figures 6 and 7); a line 19 for acquisition of an image of the reference retinal region 4a; a line 20 for acquisition of corneo-scleral references (i.e., the position of blood vessels); and a line 21 for acquisition of an image of the iris 3. A system of optical dividers 110 directs the radiation reflected by the cornea 2, by the iris 3 and by the retina 4 towards the lines 18, 19, 20 and 21.

Moreover, to obtain greater efficiency the image acquisition device 14 cooperates with the source 101, which emits in the infrared around a wavelength of λ_{IR}, and with the sources 102, 103, which emit in the visible range around respective wavelengths λ_{V1}, λ_{V2} differing from the wavelength λ_{F} of the beam F. Consequently, the band of reflected radiation emerging from the eye 1 principally contains four contributions:
a first contribution, in the visible (λ_{F}), is due to the reflection of the beam F on the retina 4 and, in practice, is an image of the retinal projection R;
a second contribution, in the infrared (λ_{IR}), is due to the light coming from the source 101 and reflected by the iris 3 (in fact, the retina 4 absorbs the infrared radiation while the iris 3 reflects it);
a third contribution, again in the visible, but at a different wavelength (λ_{V1}), is due to the light emitted by the source 102 and reflected by the retina 4; and
a fourth contribution, once again in the visible, at another different wavelength (λ_{V2}), is due to the light emitted by the source 103 and reflected by the cornea 2 and by the sclera of the eye 1 (in practice, the third and fourth contribution contain images respectively of the reference retinal region 4a and of the corneo-scleral references).

The line 18 comprises a camera 22, preferably digital, focused on the retina 4 of the eye 1. The camera 22 is provided with an optical filter 23, having a first passband centered around the wavelength λ_{F} of the laser source 6, and thus principally receives the first contribution present in the light reflected by the eye 1, forming an image of the retinal projection R.

The line 19 comprises a digital camera 24, also focused on the retina 4 and equipped with an optical filter 25. The optical filter 25 has a second passband, differing from the first passband of the optical filter 23 and centered around the wavelength λ_{V1} of the source 102; therefore, the camera 24 principally receives the contribution of light due to the reflection of the source 101 on the retina 4 (image of the reference retinal region 4a).

The line 20 comprises a digital camera 26, focused on the surface of the cornea 2. The camera 26 is provided with an optical filter 27, having a third passband, centered around the wavelength λ_{V2} of the source 103. Therefore, the camera 26 principally receives the contribution due to the light emitted by the source 103 and reflected by the cornea 2 and by the sclera of the eye 1; in particular, this contribution forms an image of the corneo-scleral reference.

The line 21 comprises a digital camera 28 focused on the iris 3. The camera 28 is provided with an optical filter 29 having a passband centered on the wavelength λ_{IR} of the source 101. Therefore, the camera 28 principally receives the contribution due to the light emitted by the source 101 and reflected by the iris 3. In particular, this contribution forms an image of the iris 3, which may be advantageously used to define the portion of the corneal surface useful for vision in pre-determined conditions of lighting (in fact, rays incident on the cornea 2 outside the useful portion are intercepted by the iris 3 and are not received by the retina 4). In practice, the optical filters 23, 25, 27 and 29 select the wavelengths suitable to favor recognition of the retinal projection R, of the reference retinal region 4a, of the corneo-scleral references and of the iris 3 respectively. In particular, each of the optical filters 23, 25, 27, 29 selects one of the contributions present in the radiation reflected by the eye 1, essentially eliminating the others; consequently, the images acquired by the cameras 22, 24, 26, 28 are free from unnecessary details, clear and precise and can thus be easily processed.

The logic control unit 15 is connected to the image acquisition device 14 and to the actuator 13 to control the angular directing device 12 on the basis of data detected by the image acquisition device 14 and operates in a closed circuit.

With reference to Figure 8, the logic control unit 15 comprises a calculation unit 30, four image processing units 31, 32, 33, 34, connected to the calculation unit 30, and to the cameras 22, 24, 26, 28 respectively, a drive unit 35 and a memory 36. The image processing unit 31 receives from the camera 22 a digital image of the retinal projection R of the beam F (figures 6 and 7) and processes it to determine the position Rxy of said retinal projection R in the plane XY.

Similarly, the image processing unit 32 receives from the camera 24 an image of the reference retinal region 4a and processes it to determine a center C of the reference retinal region 4a and the position Cxy of said center C in the plane XY (see also Figure 7).

The image processing unit 33 receives from the camera 26 a corneo-scleral image from which it extracts the position of the pre-determined corneo-scleral references in the plane XY.

The image processing unit 34 receives from the camera 28 an image of the iris 3, from which it extracts the position in the play XY of the projection of the iris 3 on the surface of the cornea 2.

The position R_{XY} of the retinal projection R, the position C_{XY} of the center C of the reference retinal region 4a, the position of the corneo-scleral references and the position of the projection of the iris 3 on the cornea 2 are supplied to the calculation unit 30.

On the basis of the signals received by the image processing units 31, 32, the calculation unit 30 determines a relative deviation S (Figures 6, 7) of the retinal projection R with respect to the center C of the reference retinal region 4a and supplies it to the drive unit 35 (the relative deviation S is a vector and is therefore provided with modulus, direction and sense); as above mentioned, said relative deviation S normally differs from zero when there are refractive defects of the eye 1. Utilizing the corneo-scleral references, the calculation unit 30 also detects any movements of the eye 1 and produces an alignment control signal CA indicative of these movements; the alignment control signal CA is supplied to the pointing device 7 and to the actuator 13 of the angular positioning device 12, which are controlled so as to realign the axis Z with the optical axis A, maintaining the direction D and the point P of incidence of the beam F on the cornea 2 constant with respect thereto.

The drive unit 35 determines, according to a known algorithm, a theoretical correction to apply to the point P of the cornea 2 to cancel the relative deviation S; for example, the drive unit 35 uses one of the algorithms employed by traditional ray-tracing systems to calculate the refractive error. In practice, applying the theoretical correction corresponds to modifying the direction D of incidence of the beam F on the point P of the cornea 2; the same effect would be obtained by fitting a lens with a dioptric power capable of focusing the beam F on the reference retinal region 4a. Therefore, for this purpose the drive unit 35 determines a new direction D' of incidence, characterized by respective updated values of the first and of the second angle of incidence α, β, and supplies to the actuator 13 a first and a second control signal Sα, Sβ, correlated to said updated values; through the first and the second control signal Sα, Sβ, the actuator 13 controls the angular positioning device 12 so as to direct the beam F on the point P of incidence of the cornea according to the new direction D' of incidence. In particular, the actuator 13 positions the mirror 16 on the basis of the first control signal Sα and the second mirror 17 on the basis of the second control signal Sβ.

As already mentioned, the theoretical correction is usually only approximate and, therefore, even after having modified the direction of incidence of the beam F a first time, the retinal projection R of the beam F may still fall outside the reference retinal region 4a, which, in the non-limiting specific case described, coincides with the fovea. In this case, the logic control unit 15 operates iteratively: in practice, a new relative deviation S is detected between the center C of the reference retinal region 4a and the retinal projection R of the beam F (the relative deviation has lower modulus than in the previous iteration) and the direction of incidence of the beam F is consequently further modified, by means of the control signals Sα, Sβ.

Therefore, in this way the logic control unit 15 controls the angular directing device 12 so as to minimize the relative deviation S between the center C of the reference retinal region 4a and the retinal projection R of the beam F incident on the point P of the cornea 2. When the relative deviation S determined by the calculation unit 30 is essentially zero, that is when the retinal projection R of the beam F falls on the reference retinal region 4a, the measurement procedure is interrupted and a final direction D_{F} of incidence of the beam is stored in the memory 36. The final direction D_{F} is indicative of the real local refractive error relative to the point P and takes account of the overall effect of all the defects present along the path of the beam F between said point P and the retinal projection R.

Upon termination of measurement of the refractive error relative to a point of the cornea 2, the beam F is directed towards a new point and measurement is repeated, until all of a pre-determined portion of the cornea 2, preferably corresponding to the projection of the iris 3 determined by the line 21, has been analyzed. Therefore, upon termination of the procedure the memory 36 contains a map of the local refractive errors, represented by the final directions of incidence of the beam F for all the points of the cornea 2 observed.

It must be pointed out that the refractometer 1 is suitable to be employed for evaluation of the refractive power and of any refractive defects of a generic lens or system of lenses and not only of the eye. In this case, the beam F is directed at a point P of a lens to be examined, similarly to what has been described in relation to the cornea 2; the image of the projection of the beam F on a screen arranged along the path of the beam F downstream of the lens is then acquired (similarly to the retinal projection R of the beam F on the retina 4) ; the acquired image is then sent to the control unit 15, to determine a deviation of the projection of the beam on the screen with respect to a pre-determined region of reference, for example selected on said screen; the control unit controls the angular directing device 12, to modify the direction of incidence of the beam F on the point P of incidence so as to minimize deviation of the projection of the beam on the screen, with respect to the region of reference.

Finally, it is evident that modifications and variants may be made to the refractometer described, without however departing from the scope of the present invention.

In the first place, some of the image acquisition lines and the corresponding image processing units of the control unit may be excluded.

In particular, in a refractometer produced according to a variant, not shown, of the invention, the line for acquisition of the image of the reference retinal region is excluded. In this case, the center of vision may advantageously be selected as the reference retinal region; alternatively, the reference retinal region may be selected by an operator arbitrarily. In fact, it must be borne in mind that convergence of the retinal projections of the rays impinging on the cornea towards any point of the retina is sufficient to guarantee acceptable vision; clearly, as the fovea has maximum sensitivity it represents a preferred, although not exclusive, point of convergence.

To determine the coordinates of the center of vision the incident beam is directed towards a point of the cornea 2 with four pre-determined distinct angulations, in succession (for example, a maximum value and a minimum value of the angle α are first set, while the angle β is zero; subsequently, the angle β is set at a maximum value and at a minimum value, while the angle α is zero). Therefore, the position of the four corresponding retinal projections is determined: the barycenter of said retinal projections coincide with the center of vision.

According to further embodiments, not shown, the line for acquisition of the corneo-scleral reference and the line for acquisition of an image of the iris may also be excluded.

Moreover, the direction of incidence of the beam on the cornea may be controlled by acting on different parameters with respect to the angles of incidence described. Furthermore, normal analog cameras associated with respective analog-digital converters may be utilized to acquire the images. It is also possible to provide a single image acquisition line, equipped with at least two filters that can be utilized alternatively, and a single image processing unit to acquire images of the retinal projection and of the center of the fovea in sequence.

## Claims

1. Refractometer, comprising a first source (6) of light, supplying a light beam (F) with an essentially spot cross section, and optical pointing means (7) of the beam (F), for directing the beam (F) towards a point (P) of incidence selectable on a cornea (2) of an eye (1) of a patient;
**characterized in that** it comprises:
image acquisition means (14) for acquiring at least a first image of a retinal projection (R) of the beam (F);
a control unit (15), connected to said image acquisition means (14), for determining a deviation (S) of the retinal projection (R) with respect to a pre-determined reference region (4a) of the eye;
optical directing means (12) of the beam (F), controlled by said control unit (15) for modifying a direction (D) of incidence of the beam (F) on the point (P) of incidence so as to minimize said deviation (S).

2. Refractometer as claimed in claim 1, **characterized in that** the control unit (15) is of the iterative type.

3. Refractometer as claimed in claim 1 or 2, **characterized in that** the control unit (15) comprises a first image processing unit (31), for determining a position (R_{XY}) of the retinal projection (R) with respect to a pre-determined reference system (X, Y, Z), on the basis of the first image.

4. Refractometer as claimed in claim 3, **characterized in that** the control unit (15) comprises a second image processing unit (32), for determining a position (C_{XY}) of the reference region (4a) with respect to said reference system (X, Y, Z), on the basis of at least a second image acquired by the image acquisition means (14).

5. Refractometer as claimed in claim 4, **characterized in that** the control unit (15) comprises a calculation unit (30), connected to the first and to the second image processing unit (31, 32) to determine said deviation (S), on the basis of the position (R_{XY}) of the retinal projection (R) and of the position (C_{XY}) of the reference region (4a) with respect to said reference system (X, Y, Z).

6. Refractometer as claimed in claim 4 or 5, **characterized in that** said image acquisition means (14) comprise a first image acquisition line (18), for acquiring said first image of the retinal projection (R), the first image acquisition line (18) being connected to the first image processing unit (31).

7. Refractometer as claimed in claim 6, **characterized in that** the beam (F) has a first wavelength (λ_{F}) and **in that** said first image acquisition line (18) comprises a first camera (22), connected to the first image processing unit (31) and equipped with a first optical filter (23) having a first passband centered around the first wavelength (λ_{F}).

8. Refractometer as claimed in claim 7, **characterized in that** said image acquisition means (14) comprise a second image acquisition line (19), for acquiring said second image of the reference region (4a), the second image acquisition line (19) being connected to the second image processing unit (32).

9. Refractometer as claimed in claim 8, **characterized in that** it comprises an auxiliary second source (102) of visible light having a second wavelength (λ_{V1}) and **in that** said second image acquisition line (19) comprises a second camera (24), connected to the second image processing unit (32) and equipped with a second optical filter (25) having a second passband, different from the first passband of the first optical filter (23) and centered around the second wavelength (λᵥ₁).

10. Refractometer as claimed in any one of claims 3 to 9, **characterized in that** said image acquisition means (14) comprise a third image acquisition line (20), for acquiring a third image of the cornea (2), and **in that** the control unit (15) comprises a third image processing unit (33), connected to the third image acquisition line (20), to determine a position of the cornea (2) with respect to said reference system (X, Y, Z).

11. Refractometer as claimed in claim 10, **characterized in that** it comprises an auxiliary third source (103) of visible light having a third wavelength (λ_{V2}) and **in that** said third image acquisition line (20) comprises a third camera (26) provided with a third optical filter (27), having a third passband centered around the third wavelength (λ_{V2}).

12. Refractometer as claimed in any one of claims 3 to 11, **characterized in that** said image acquisition means (14) comprise a fourth image acquisition line (21), for acquiring a fourth image of an iris (3) of the eye (1), and **in that** the control unit (15) comprises a fourth image processing unit (34) connected to the fourth image acquisition line (21), to determine a position of the iris (3) with respect to said reference system (X, Y, Z).

13. Refractometer as claimed in claim 14, **characterized in that** it comprises an auxiliary fourth source (101) of infrared light having a fourth wavelength (λ_{IR}) and **in that** said fourth image acquisition line (21) comprises a fourth camera (28) provided with a fourth optical filter (29), having a fourth passband centered around the fourth wavelength (λ_{IR}).

14. Refractometer as claimed in any one of the previous claims, **characterized in that** the control unit (15) comprises memory means (36) for storing a final direction (D_{F}) of incidence of the beam (F) on the point (P) of incidence when said deviation (S) is essentially zero.

15. Refractometer as claimed in any one of the previous claims, **characterized in that** the control unit (15) comprises a drive unit (35), connected to said directing means (12) of the beam (F), to supply the optical directing means (12) with control signals (Sα, Sβ) correlated to said deviation (S) and **in that** said optical directing means (12) comprise a first and a second deflection mirror (16, 17), positionable to modify a first and, respectively, a second angle of incidence (α, β) of the beam (F) on the cornea (2).

16. Control method of a refractometer comprising the steps of:
activating a light source (6), to produce a light beam (F) having an essentially spot cross section; and
controlling pointing means (7) of the light beam (F), to direct the light beam (F) towards a point (P) of incidence selectable on a cornea (2) of an eye (1) of a patient;
**characterized in that** it comprises the steps of:
acquiring at least a first image of a retinal projection (R) of the beam (F);
supplying said first image to a control unit (15), to determine a deviation (S) of the retinal projection (R) with respect to a pre-determined reference region (4a) of the eye;
controlling optical directing means (12) of the beam (F), for modifying a direction (D) of incidence of the beam (F) on the point (P) of incidence so as to minimize said deviation (S).

17. Method as claimed in claim 16, **characterized in that** the step of controlling optical directing means (12) is repeated iteratively, until said deviation (S) is essentially cancelled.

18. Method as claimed in claim 16 or 17, **characterized in that** said first image of the retinal projection (R) is processed to determine a first position (R_{XY}) of the retinal projection (R) with respect to a pre-determined reference system (X, Y, Z).

19. Method as claimed in claim 18, **characterized in that** a second image is acquired and processed to determine a second position (C_{XY}) of the reference region (4a) with respect to said reference system (X, Y, Z).

20. Method as claimed in claim 19, **characterized in that** said first image is filtered with a first passband and said second image is filtered with a second passband, different from said first passband.

21. Method as claimed in any one of claims 16 to 20, **characterized in that** a final direction (D_{F}) of incidence of the beam (F) on the point (P) of incidence is stored, when said deviation (S) is essentially zero.

22. Method as claimed in any one of claims 16 to 21, **characterized in that** the step to control optical directing means (12) comprises positioning a first and a second deflection mirror (16, 17) to modify a first and, respectively, a second angle of incidence (α, (β) of the beam (F) on the cornea (2).

23. Control method of a refractometer comprising the steps of:
activating a light source (6), to produce a light beam (F) having an essentially spot cross section; and
controlling pointing means (7) of the light beam (F), to direct the light beam (F) towards a point (P) of incidence selectable on a lens (2);
**characterized in that** it comprises the steps of:
acquiring at least a first image of a projection (R) of the beam (F) on a screen (3), positioned along the path of the beam downstream of the lens (2);
supplying said first image to a control unit (15), for determining a deviation (S) of the projection (R) with respect to a pre-determined reference region (4a);
controlling optical positioning means (12) of the beam (F), for modifying a direction (D) of incidence of the beam (F) on the point (P) of incidence so as to minimize said deviation (S).
